# EUROPEAN PATENT SPECIFICATION

(11) **EP 1 728 799 B1**
(45) Date of publication and mention of the grant of the patent: **20.08.2008**
(21) Application number: 05011950.2
(22) Date of filing: 02.06.2005
(51) Int. Cl.: C07K 14/00

(54) **Human kinase interacting protein 2 (KIP2)-related gene variant (KIP2V1) associated with prostate cancer**
Human Kinase interacting Protein 2 (KIP2)-related gene variant (KIP2V1) im Zusammenhang mit Prostatakrebs
Human Kinase interacting Protein 2 (KIP2)-related gene variant (KIP2V1) associée au cancer de la prostate

(43) Date of publication of application: 06.12.2006
(73) Proprietor: Bioptik Technology, Inc., Hsinchu (TW)
(72) Inventor: Dai, Ken-Shwo, Industrial Park, Hsinchu (TW)
(74) Representative: Becker Kurig Straus

(56) References cited:
- US-A1- 2004 259 086
- LODYGIN DIMITRI ET AL: "Functional epigenomics identifies genes frequently silenced in prostate cancer" CANCER RESEARCH, vol. 65, no. 10, May 2005 (2005-05), pages 4218-4227, XP002344096 ISSN: 0008-5472
- DATABASE Geneseq [Online] 26 February 2004 (2004-02-26), "Novel human secreted and transmembrane protein PRO182." XP002344102 retrieved from EBI accession no. GSP:ADG24809 Database accession no. ADE24809 & US 2003/190622 A1 (DAI KEN-SHWO) 9 October 2003 (2003-10-09)
- DATABASE Geneseq [Online] 26 February 2004 (2004-02-26), "Novel human secreted and transmembrane protein PRO182 cDNA." XP002344103 retrieved from EBI accession no. GSN:ADG24808 Database accession no. ADE24808 & US 2003/190622 A1 (DAI KEN-SHWO) 9 October 2003 (2003-10-09)
- DATABASE Geneseq [Online] 21 October 2004 (2004-10-21), "Breast cancer prognosis marker #1276." XP002344104 retrieved from EBI accession no. GSN:ADR25415 Database accession no. ADR25415

## Description

### FIELD OF THE INVENTION

The invention relates to the nucleic acid of a novel human kinase interacting protein 2 (KIP2)-related gene variant (KIP2V1), the polypeptide encoded thereby, the preparation process thereof, and the uses of the same in diagnosing diseases associated with the presence of KIP2V1, in particular, prostate cancer.

### BACKGROUND OF THE INVENTION

Prostate cancer is the second leading cause of cancer death in men. The risk of prostate cancer is increased with age. Incidence of prostate cancer was estimated to be doubled by the year 2030 (Boyle, (1996) Eur Urol. 29 Suppl 2:3-9). In recent years, much progress has been made toward understanding the molecular and cellular biology of prostate cancer. Many important contributions have been made by the identification of several key genetic factors associated with prostate cancer (Gimba and Barcinski, (2003) Int Braz J Urol. 29:401-410). However, there are still some limitations in the use of biological markers for detecting prostate cancer (Lindstedt et al., (1990) Clin Chem. 36(1):53-58; Stamey et al., (1987) N Engl J Med. 317(15):909-16; Oesterling et al., (1988) J Urol. 139(4):766-772), suggesting that identification of new markers for diagnosing prostate cancer is needed.

Recently, many factors associated with prostate cancer have been reported (Gimba and Barcinski, (2003) Int Braz J Urol. 29:401-410). One of the contributing factors is the gene(s) associated with cell cycle control. Thus, future strategies for the prevention and treatment of prostate cancer will be focused on identification, isolation and elucidation of genes involved in cell cycle regulation. A human kinase interacting protein 2 (KIP2) gene, candidate tumor suppressor gene (Matsuoka et al. (1995) Genes Dev 9:650-62), is a member of Cip/Kip family involved in the negative regulation of the cell cycle at the G1 checkpoint (Lee et al. (1995) Genes Dev 9:639-49; Harper and Elledge, (1996) Curr Opin Genet Dev 6:56-64; Sherr, (1996) Science 274:1672-7; Lee and Yang, (2001) Cell Mol Life Sci 58:1907-22). The tumor suppressor activity of KIP2 was shown to be mediated via its interaction with cyclin A-CDK2 (Adkins and Lumb, (2002) Proteins 46:1-7) or proliferating cell nuclear antigen (PCNA; Watanabe et al. (1998) Proc Natl Acad Sci USA 95:1392-1397). Overexpression of KIP2 was reported to arrest cells in Gl (Lee et al. (1995) Genes Dev 9:639-49; Matsuoka et al. (1995) Genes Dev 9:650-62). Decreased expression of KIP2 has been observed in many cancers (Chung et al. (1996) Hum Mol Genet 5:1101-8; Oya and Schulz, (2000) Br J Cancer 83:626-31; Dauphinot et al. (2001) Oncogene 20:3258-65; Ito et al. (2001) Oncology 61:221-5). These findings strongly implicate that KIP2 may have a role in the tumorigenic process of prostate cancer. Therefore, the discovery of gene variants of KIP2 from prostate cancer may be important targets for diagnostic markers of prostate cancer.

US20030190622 A1 discloses a KIP2-related gene variant (KIP2V) and its use in diagnosing lung cancers. It does not disclose or suggest any other KIP2-related variants.

### SUMMARY OF THE INVENTION

The present invention provides a KIP2-related gene variant (KIP2V1) present in human prostate cancer. The nucleotide sequence of KIP2V1 and the polypeptide sequence encoded thereby can be used for the diagnosis of diseases associated with the presence of KIPV1, in particular, prostate cancer.

The invention further provides an expression vector and host cell for expressing KIP2V1.

The invention further provides a method for producing the polypeptide of KIP2V1.

The invention also provides methods for diagnosing diseases associated with the presence of KIP2V1, in particular, prostate cancer.

The invention also provides kits for diagnosing diseases associated with the presence of KIP2V1, in particular, prostate cancer.

### BRIEF DESCRIPTION OF THE DRAWINGS

Figs. 1A and 1B show the RT-PCR analysis of KIP2V1 and GAPDH (internal control) expression, respectively, in 27 cell lines.

Figs. 2A to 2D show the nucleotide sequence alignment of human KIP2, KIP2V (SEQ ID NO. 1), and KIP2V1 (SEQ ID NO. 3) genes and two cDNA fragments 235bp (LF) and 203bp (SF) obtained from RT-PCR analysis of Fig 1A.

Fig. 3 shows the amino acid sequence alignment of human KIP2 protein and the polypeptides of KIP2V (SEQ ID NO: 2) and KIP2V1 (SEQ ID NO: 4)

### DETAILED DESCRIPTION OF THE INVENTION

According to the present invention, all technical and scientific terms used have the same meanings as commonly understood by persons skilled in the art.

The term "antibody," as used herein, denotes intact molecules (a polypeptide or group of polypeptides) as well as fragments thereof, such as Fab, R(ab')₂, and Fv fragments, which are capable of binding the epitopic determinutesant. Antibodies are produced by specialized B cells after stimulation by an antigen. Structurally, antibody consists of four subunits including two heavy chains and two light chains. The internal surface shape and charge distribution of the antibody binding domain are complementary to the features of an antigen. Thus, antibody can specifically act against the antigen in an immune response.

The term "base pair (bp)," as used herein, denotes nucleotides composed of a purine on one strand of DNA which can be hydrogen bonded to a pyrimidine on the other strand. Thymine (or uracil) and adenine residues are linked by two hydrogen bonds. Cytosine and guanine residues are linked by three hydrogen bonds.

The term "Basic Local Alignment Search Tool (BLAST; Altschul et al., (1997) Nucleic Acids Res. 25: 3389-3402)," as used herein, denotes programs for evaluation of homologies between a query sequence (amino or nucleic acid) and a test sequence as described by Altschul et al. (Nucleic Acids Res. 25: 3389-3402, 1997). Specific BLAST programs are described as follows:

(1) BLASTN compares a nucleotide query sequence against a nucleotide sequence database;

(2) BLASTP compares an amino acid query sequence against a protein sequence database;

(3) BLASTX compares the six-frame conceptual translation products of a query nucleotide sequence against a protein sequence database;

(4) TBLASTN compares a query protein sequence against a nucleotide sequence database translated in all six reading frames; and

(5) TBLASTX compares the six-frame translations of a nucleotide query sequence against the six-frame translations of a nucleotide sequence database.

The term "cDNA," as used herein, denotes nucleic acids that synthesized from a mRNA template using reverse transcriptase.

The term "cDNA library," as used herein, denotes a library composed of complementary DNAs which are reverse-transcribed from mRNAs.

The term "complement," as used herein, denotes a polynucleotide sequence capable of forming base pairing with another polynucleotide sequence. For example, the sequence 5'-ATGGACTTACT-3' (SEQ ID NO: 5) binds to the complementary sequence 5'- AGTAAGTCCAT-3' (SEQ ID NO: 6).

The term "deletion," as used herein, denotes a removal of a portion of one or more amino acid residues/nucleotides from a gene.

The term "expressed sequence tags (ESTs)," as used herein, denotes short (200 to 500 base pairs) nucleotide sequence that derives from either 5' or 3' end of a cDNA.

The term "expression vector," as used herein, denotes nucleic acid constructs which contain a cloning site for introducing the DNA into vector, one or more selectable markers for selecting vectors containing the DNA, an origin of replication for replicating the vector whenever the host cell divides, a terminator sequence, a polyadenylation signal, and a suitable control sequence which can effectively express the DNA in a suitable host. The suitable control sequence may include promoter, enhancer and other regulatory sequences necessary for directing polymerases to transcribe the DNA.

The term "host cell," as used herein, denotes a cell which is used to receive, maintain, and allow the reproduction of an expression vector comprising DNA. Host cells are transformed or transfected with suitable vectors constructed using recombinant DNA methods. The recombinant DNA introduced with the vector is replicated whenever the cell divides.

The term "insertion" or "addition," as used herein, denotes the addition of a portion of one or more amino acid residues/nucleotides to a gene.

The term "in silico," as used herein, denotes a process of using computational methods (e.g., BLAST) to analyze DNA sequences.

The term "polymerase chain reaction (PCR)," as used herein, denotes a method which increases the copy number of a nucleic acid sequence using a DNA polymerase and a set of primers (about 20bp oligonucleotides complementary to each strand of DNA) under suitable conditions (successive rounds of primer annealing, strand elongation, and dissociation).

The term "protein" or "polypeptide," as used herein, denotes a sequence of amino acids in a specific order that can be encoded by a gene or by a recombinant DNA. It can also be chemically synthesized.

The term "nucleic acid sequence" or "polynucleotide," as used herein, denotes a sequence of nucleotide (guanine, cytosine, thymine or adenine) in a specific order that can be a natural or synthesized fragment of DNA or RNA. It may be single-stranded or double-stranded.

The term "reverse transcriptase-polymerase chain reaction (RT-PCR)," as used herein, denotes a process which transcribes mRNA to complementary DNA strand using reverse transcriptase followed by polymerase chain reaction to amplify the specific fragment of DNA sequences.

The term "transformation," as used herein, denotes a process describing the uptake, incorporation, and expression of exogenous DNA by prokaryotic host cells.

The term "transfection," as used herein, a process describing the uptake, incorporation, and expression of exogenous DNA by eukaryotic host cells.

The term "variant," as used herein, denotes a fragment of sequence (nucleotide or amino acid) inserted or deleted by one or more nucleotides/amino acids.

In the first aspect, the present invention provides the nucleic acid sequence of the novel human KIP2-related gene variant (KIP2V1) as well as the polypeptide of KIP2V1.

According to the present invention, a cDNA clone, named KIP2V1 (KIP2 gene variant 1), was isolated from a prostate carcinoma (DU145 cell line) cDNA library in a RT-PCR analysis, and then was sequenced.

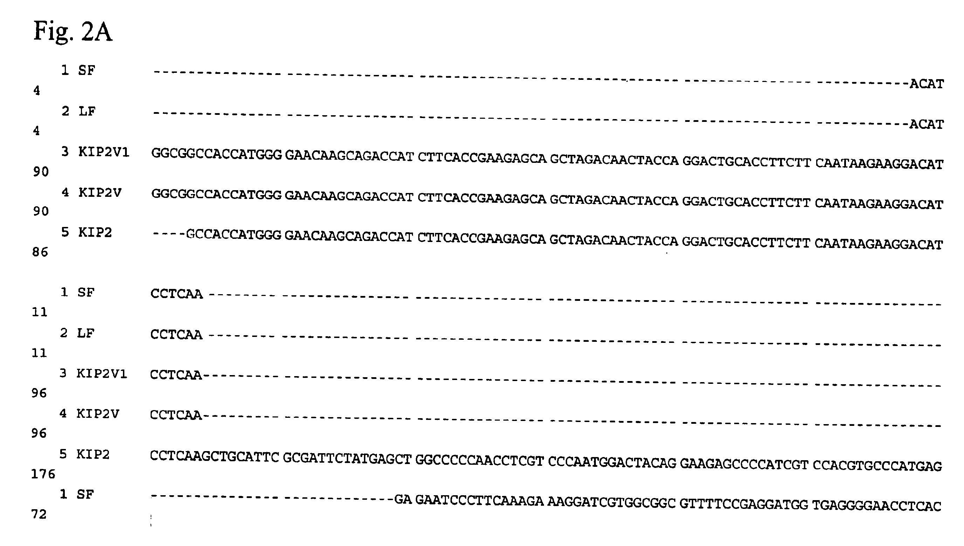

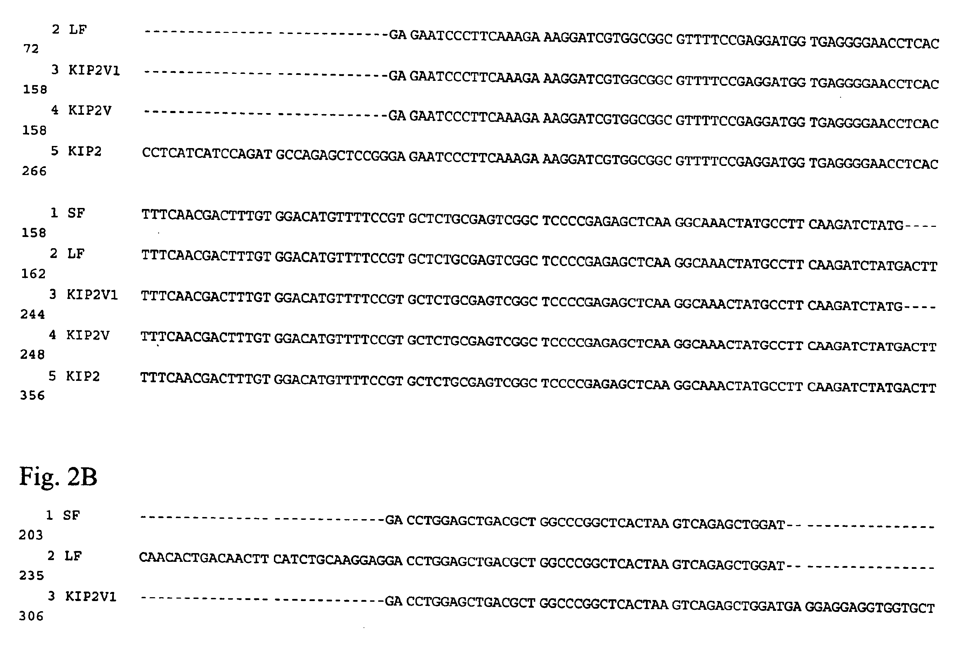

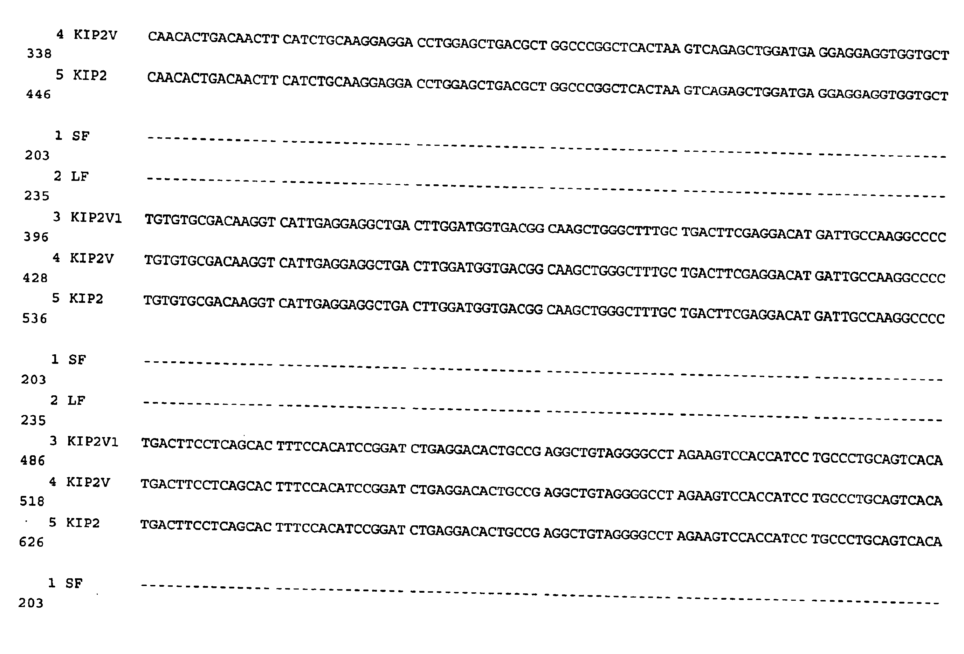

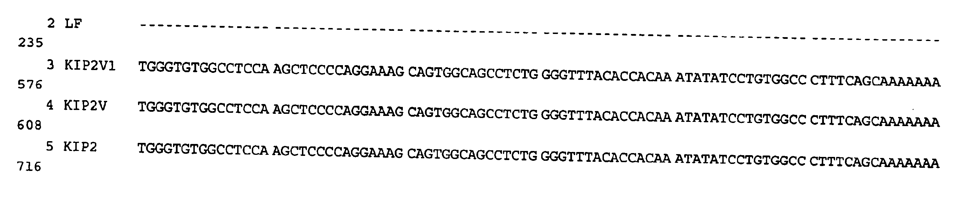

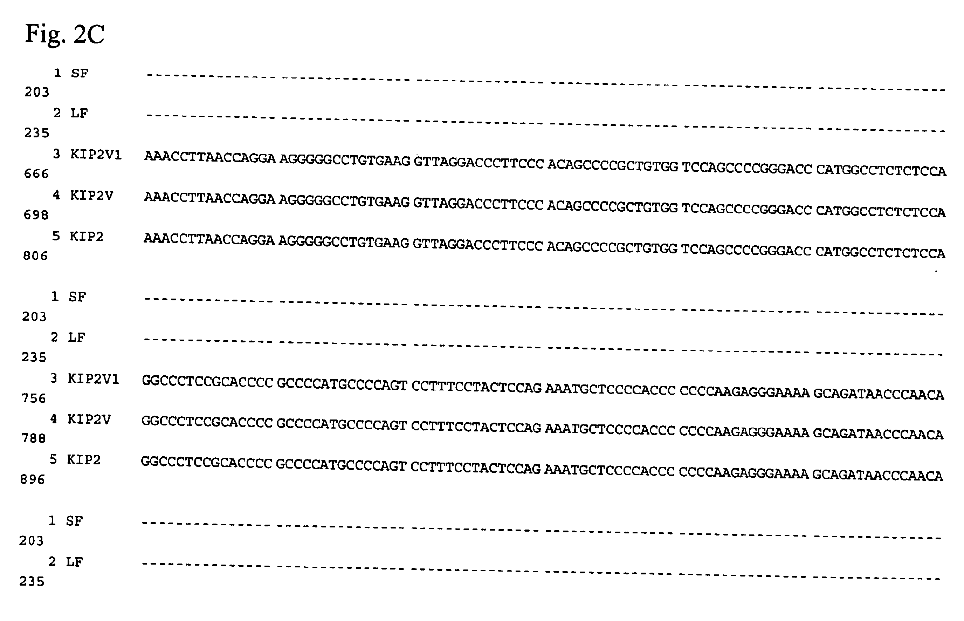

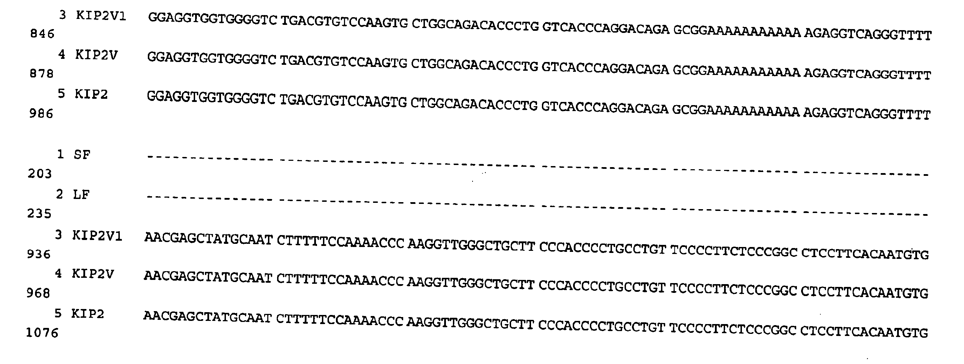

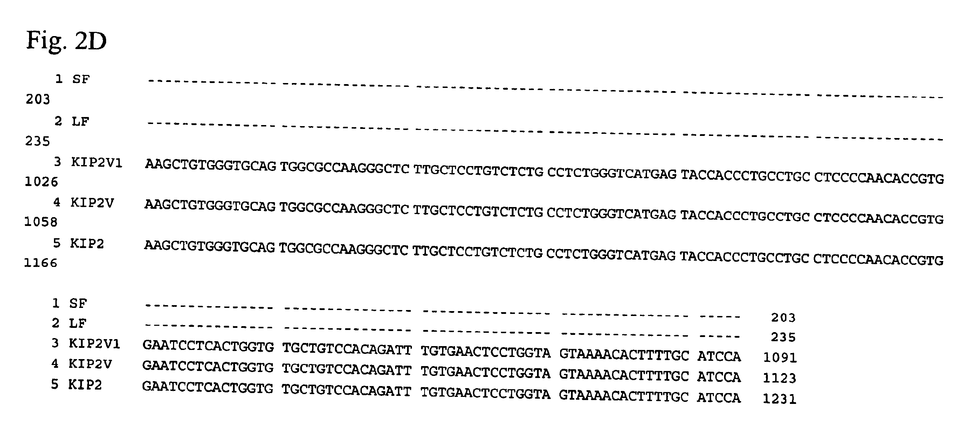

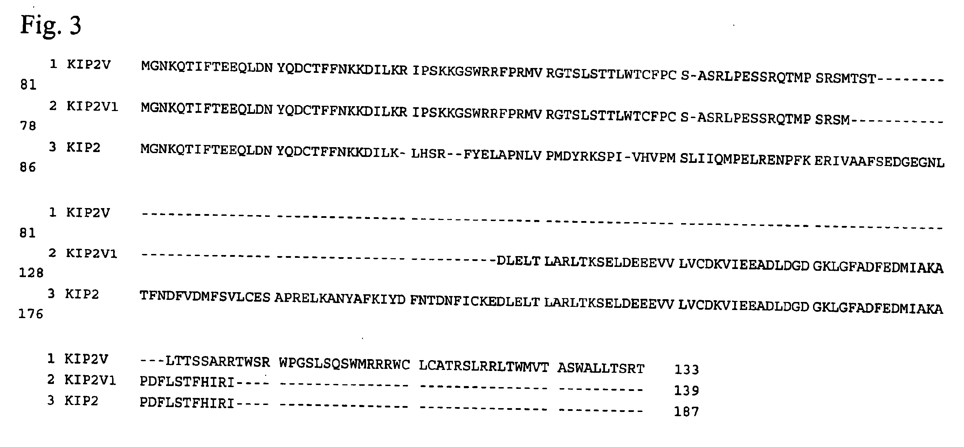

The full-length of the KIP2V1 cDNA is a 1091bp clone (SEQ ID NO: 3) containing a 416bp open reading frame (ORF) extending from 11bp to 427bp, which corresponds to an encoded protein of 139 amino acid residues (SEQ ID NO: 4) with a predicted molecular mass of 15.9 kDa. The sequence around the initiation ATG codon of KIP2V1 (located at nucleotide 11 to 13bp) was matched to the Kozak consensus sequence (A/GCCATGG) (Kozak, (1987) Nucleic Acids Res. 15: 8125-48; Kozak, (1991) J Cell Biol. 115: 887-903.). To determine the variation in sequence of KIP2V1 cDNA clone, an alignment of the nucleotide sequences of KIP2 with KIP2V, KIP2V1 and two RT-PCR fragments [a long fragment (LF; 235bp) and a short fragment (SF; 203bp)] was performed (Fig. 2A to 2D). An alignment of the amino acid sequences of KIP2 with KIP2V and KIP2V1 was also performed (Figs. 3A to 3B). Two major genetic deletions were found in the nucleic acid sequence of KIP2V1 as compared to that of KIP2. One 112bp deletion is located in the sequence of KIP2 from nucleotides 94 to 205 and the other 32bp deletion is located in the sequence of KIP2 from nucleotides 352 to 385. It is also found that 1) the N-terminal section (the first 29 amino acids) of the predicted amino acid sequence of KIP2V1 (SEQ ID NO: 4) is conserved with those of KIP2 and KIP2V; 2) the amino acid sequence (30-78 amino acids) of KIP2V1 is conserved with that of KIP2V (30-78 amino acids) but different from that of KIP2; 3) the amino acid sequence (79-139 amino acids) of KIP2V1 is conserved with the amino acid sequence (127-187 amino acids) of KIP2 but different from that of KIP2V.

In the present invention, a search of ESTs deposited in dbEST (Boguski et al. (1993) Nat Genet. 4: 332-3) at National Center for Biotechnology Information (NCBI) was performed to determine the tissue distribution of KIP2V1 in silico. The result of in silico Northern analysis showed that no EST was found to confirm the absence of 32bp region on KIP2V1 nucleotide sequence suggesting that the nucleotide fragment comprising nucleotides 244 to 245 of KIP2V1 is a novel cDNA fragment and can be used as a probe for determining the presence of KIP2V1 under high stringency conditions. An alternative approach is that any set of primers for amplifying the fragment containing nucleotides 244 to 245 of KIP2V1 may be used for determining the presence of the variant.

Scanning the KIP2V1 sequence against the profile entries in PROSITE (ScanProsite) indicated that KIP2V1 protein contains three protein kinase C phosphorylation sites (33-35aa, 37-39aa and 68-70aa), one cAMP- and cGMP-dependent protein kinase phosphorylation site (34-37aa), two N-myristoylation sites (2-7aa and 47-52aa), one Tyrosine sulfation site (9-23aa), two Casein kinase II phosphorylation site (88-91aa and 91-93 aa) and one EF-hand calcium-binding domain (109-121aa).

According to the present invention, the polypeptide of KIP2V1 and its fragment thereof may be produced through genetic engineering techniques. For instance, they may be produced by using appropriate host cells which have been transformed with recombinant DNAs that code for the desired polypeptides or fragments thereof. The nucleotide sequence of KIP2V1 or the fragments thereof is inserted into an appropriate expression vector, i.e., a vector which contains the necessary elements for the transcription and translation of the inserted coding sequence in a suitable host. The nucleotide sequence is inserted into the vector in a manner that it will be expressed under appropriate conditions (e.g., in proper orientation and correct reading frame and with appropriate expression sequences, including an RNA polymerase binding sequence and a ribosomal binding sequence).

Any method that is known to those skilled in the art may be used to construct expression vectors containing the sequence of KIP2V1 and appropriate transcriptional/translational control elements. These methods may include in vitro recombinant DNA and synthetic techniques, and in vivo genetic recombinant techniques. (See, e.g., Sambrook, J. Cold Spring Harbor Press, Plainview N.Y., Ch. 4, 8, and 16-17; Ausubel, R. M. et al. (1995) Current protocols in Molecular Biology, John Wiley & Sons, New York N.Y., ch. 9, 13, and 16.)

A variety of expression vector/host systems may be utilized to express KIP2V1. These include, but not limited to, microorganisms such as bacteria transformed with recombinant bacteriophage, plasmid, or cosmid DNA expression vector; yeast transformed with yeast expression vector; insect cell systems infected with virus (e.g., baculovirus); plant cell system transformed with viral expression vector (e.g., cauliflower mosaic virus, CaMV, or tobacco mosaic virus, TMV); or animal cell system infected with virus (e.g., vaccina virus, adenovirus, etc.). Preferably, the host cell is a bacterium, and more preferably, the bacterium is E. coli.

Alternatively, the polypeptide of KIP2V1 or fragments thereof may be synthesized using chemical methods. For example, peptide synthesis can be performed using various solid-phase techniques (Roberge, J. Y. et al. (1995) Science 269: 202 to 204). Automated synthesis may be achieved using the ABI 431A peptide synthesizer (Perkin-Elmer).

According to the present invention, the fragments of the nucleotide sequence of KIP2V1 and the polypeptide encoded thereby are used as primers or probes and immunogens, respectively. Preferably, the purified fragments are used. The fragments may be produced by enzyme digestion, chemical cleavage of isolated or purified polypeptide or nucleotide sequences, or chemical synthesis and then may be isolated or purified. Such isolated or purified fragments of the polypeptides and nucleotide sequences can be used directed as immunogens and primers or probes, respectively.

Immunization of mammals with immunogens described herin, preferably humans, rabbits, rats, mice, sheep, goats, cows, or horses, is performed by following the procedures well known to those skilled in the art, for the purpose of obtaining antisera containing polyclonal antibodies or hybridoma lines secreting monoclonal antibodies.

Monoclonal antibodies can be prepared by standard techniques, given the teachings contained herein. Such techniques are disclosed, for example, in U.S. Patent Nos. 4,271,145 and 4,196,265. Briefly, an animal is immunized with the immunogen. Hybridomas are prepared by fusing spleen cells from the immunized animal with myeloma cells. The fusion products are screened for those producing antibodies that bind to the immunogen. The positive hybridoma clones are isolated, and the monoclonal antibodies are recovered from those clones.

Immunization regimens for production of both polyclonal and monoclonal antibodies are well-known in the art. The immunogen may be injected by any of a number of routes, including subcutaneous, intravenous, intraperitoneal, intradermal, intramuscular, mucosal, or a combination thereof. The immunogen may be injected in soluble form, aggregate form, attached to a physical carrier, or mixed with an adjuvant, using methods and materials well-known in the art. The antisera and antibodies may be purified using column chromatography methods well known to those skilled in the art.

Antibody fragments which contain specific binding sites for the polypeptides or fragments thereof may also be generated. For example, such fragments include, but are not limited to, F(ab')2 fragments produced by pepsin digestion of the antibody molecule and Fab fragments generated by reducing the disulfide bridges of the F(ab')2 fragments.

Many gene variants have been found to be associated with diseases (Stallings-Mann et al., (1996) Proc Natl Acad Sci U S A 93: 12394-9; Liu et al., (1997) Nat Genet 16:328-9; Siffert et al., (1998) Nat Genet 18: 45 to 8; Lukas et al., (2001) Cancer Res 61: 3212 to 9). Since KIP2V1 clone was found to be expressed in prostate cancer, it is advisable that KIP2V1 may be served as a marker for the diagnosis of prostate cancer. Thus, the expression level of KIP2V1 relative to KIP2 may be a useful indicator for screening of patients suspected of having such disease, and the index of relative expression level (mRNA or protein) may confer an increased susceptibility to the same.

Accordingly, the subject invention further provides methods for diagnosing the diseases associated with the presence of human KIP2V1 gene in a mammal, in particular, prostate cancer.

The method for diagnosing the diseases associated with prostate cancer may be performed by detecting the nucleotide sequence of the KIP2V1 of the invention, which comprises the steps of: (1) extracting the total RNA of cells obtained from the mammal; (2) amplifying the RNA by reverse transcriptase-polymerase chain reaction (RT-PCR) with a set of primers to obtain a cDNA comprising the fragments comprising nucleotides 242 to 247 of SEQ ID NO: 3; and (3) detecting whether the cDNA is obtained. If necessary, the amount of the obtained cDNA sample may be determined.

In this embodiment, one of the primers may be designed to have a sequence comprising the nucleotides of SEQ ID NO: 3 containing nucleotides 242 to 247, and the other may be designed to have a sequence complementary to the nucleotides of SEQ ID NO: 3 at any other locations downstream of nucleotide 247. Alternatively, one of the primers may be designed to have a sequence complementary to the nucleotides of SEQ ID NO: 3 containing nucleotides 242 to 247, and the other may be designed to have a sequence comprising the nucleotides of SEQ ID NO: 3 at any other locations upstream of nucleotide 242. In this case, only KIP2V1 will be amplified.

Alternatively, one of the primers may be designed to have a sequence comprising the nucleotides of SEQ ID NO: 3 upstream of nucleotide 242 and the other may be designed to have a sequence complementary to the nucleotides of SEQ ID NO: 3 downstream of nucleotide 247. In this case, both KIP2 and KIP2V1 will be amplified. The length of the PCR fragment from KIP2V1 will be 144bp shorter than that from KIP2. If both KIP2V and KIP2V1 are amplified, the length of the PCR fragment from KIP2V1 will be 32bp shorter than that from KIP2V.

The primer of the present invention contains 6 to 50 nucleotides, preferably 10 to 40 nucleotides, more preferably 15 to 30 nucleotides.

Total RNA may be isolated from patient samples by using TRIZOL reagents (Life Technology). Tissue samples (e.g., biopsy samples) are powdered under liquid nitrogen before homogenization. RNA purity and integrity are assessed by absorbance at 260/280 nm and by agarose gel electrophoresis. The set of primers designed to amplify the expected sizes of specific PCR fragments of KIP2V1 can be used. PCR fragments are analyzed on a 1% agarose gel using five microliters (10%) of the amplified products. To determine the expression levels for each gene variants, the intensity of the PCR products may be determined by using the Molecular Analyst program (version 1.4.1; Bio-Rad).

The RT-PCR experiment may be performed according to the manufacturer instructions (Boehringer Mannheim). A 50µl reaction mixture containing 2µl total RNA (0.1µg/µl), 1µl each primer (20 pM), 1µl each dNTP (10 mM), 2.5 µl DTT solution (100 mM), 10 µl 5X RT-PCR buffer, 1µl enzyme mixture, and 28.5 µl sterile distilled water may be subjected to the conditions such as reverse transcription at 60°C for 30 minutes followed by 35 cycles of denaturation at 94°C for 2 minutes, annealing at 60°C for 2 minutes, and extension at 68°C for 2 minutes. The RT-PCR analysis may be repeated twice to ensure reproducibility, for a total of three independent experiments.

Another embodiment of the method for diagnosing prostate cancer may be performed by detecting the nucleotide sequence of KIP2V1, which comprises the steps of: (1) extracting total RNA from a sample obtained from the mammal; (2) amplifying the RNA by reverse transcriptase-polymerase chain reaction (RT-PCR) to obtain a cDNA sample; (3) bringing the cDNA sample into contact with the nucleic acid encoding KIP2V1 or a nucleic acid fragment comprising nucleotides 242 to 247 of SEQ ID NO: 3; and (4) detecting whether the cDNA sample hybridizes with the nucleic acid encoding KIP2V1 or the nucleic acid fragment comprising nucleotides 242 to 247 of SEQ ID NO: 3. If necessary, the amount of the hybridized sample may be determined.

The expression of gene variants can also be analyzed using Northern blot hybridization approach. Specific fragments comprising nucleotides 242 to 247 of KIP2V1 may be amplified by polymerase chain reaction (PCR) using primer set designed for RT-PCR. The amplified PCR fragment contains 6 to 50 nucleotides, preferably 10 to 40 nucleotides, more preferably 15 to 30 nucleotides, and may be labeled and serve as a probe to hybridize the membranes containing total RNAs extracted from the samples under the conditions of 55°C in a suitable hybridization solution for 3 hr. Blots may be washed twice in 2 x SSC, 0.1% SDS at room temperature for 15 minutes each, followed by two washes in 0.1 x SSC and 0.1% SDS at 65°C for 20 minutes each. After these washes, blots may be rinsed briefly in suitable washing buffer and incubated in blocking solution for 30 minutes, and then incubated in suitable antibody solution for 30 minutes. Blots may be washed in washing buffer for 30 minutes and equilibrated in suitable detection buffer before detecting the signals. Alternatively, the presence of gene variants (cDNAs or PCR) can be detected using microarray approach. The cDNAs or PCR products corresponding to the nucleotide sequences of the invention may be immobilized on a suitable substrate such as a glass slide. Hybridization can be preformed using the labeled mRNAs extracted from samples. After hybridization, nonhybridized mRNAs are removed. The relative abundance of each labeled transcript, hybridizing to a cDNA/PCR product immobilized on the microarray, can be determined by analyzing the scanned images.

According to the invention, the method for diagnosing prostate cancer may also be performed by detecting the polypeptide encoded by the KIP2V1 of the invention. For instance, the polypeptide in protein samples obtained from the mammal may be determined by, but not limited to, the immunoassay wherein the antibodies specifically binding to the polypeptides of the invention is contacted with the sample, and the antibody-polypeptide complex is detected. If necessary, the amount of antibody-polypeptide complex can be determined.

The polypeptide encoded by KIP2V 1 may be expressed in prokaryotic cells by using suitable prokaryotic expression vectors. The cDNA fragments of KIP2V1 may be PCR-amplified using primer set with restriction enzyme digestion sites incorporated in the 5' and 3' ends, respectively. The PCR products can then be enzyme digested, purified, and inserted into the corresponding sites of prokaryotic expression vector in-frame to generate recombinant plasmids. Sequence fidelity of this recombinant DNA can be verified by sequencing. The prokaryotic recombinant plasmids may be transformed into host cells (e.g., E. coli BL21 (DE3)). Recombinant protein synthesis may be stimulated by the addition of 0.4 mM isopropylthiogalactoside (IPTG) for 3h. The bacterially-expressed proteins may be purified.

The polypeptide encoded by the KIP2V1 may be expressed in animal cells by using eukaryotic expression vectors. Cells may be maintained in Dulbecco's modified Eagle's medium (DMEM) supplemented with 10% fetal bovine serum (FBS; Gibco BRL) at 37oC in a humidified 5% CO2 atmosphere. Before transfection, the nucleotide sequence of each of the gene variant may be amplified with PCR primers containing restriction enzyme digestion sites and ligated into the corresponding sites of eukaryotic expression vector in-frame. Sequence fidelity of this recombinant DNA can be verified by sequencing. The cells may be plated in 12-well plates one day before transfection at a density of 5 x 104 cells per well. Transfections may be carried out using Lipofectaminutese Plus transfection reagent according to the manufacturer's instructions (Gibco BRL). Three hours following transfection, medium containing the complexes may be replaced with fresh medium. Forty-eight hours after incubation, the cells may be scraped into lysis buffer (0.1 M Tris HCl, pH 8.0, 0.1% Triton X-100) for purification of expressed proteins. After these proteins are purified, monoclonal antibodies against these purified proteins may be generated using hybridoma technique according to the conventional methods (de StGroth and Scheidegger, (1980) J Immunol Methods 35:1-21; Cote et al. (1983) Proc Natl Acad Sci U S A 80: 2026-30; and Kozbor et al. (1985) J Immunol Methods 81:31-42).

According to the invention, the presence of the polypeptide encoded by KIP2V1 in samples of normal prostate and prostate cancer may be determined by, but not limited to, Western blot analysis. Proteins extracted from samples may be separated by SDS-PAGE and transferred to suitable membranes such as polyvinylidene difluoride (PVDF) in transfer buffer (25 mM Tris-HCl, pH 8.3, 192 mM glycine, 20% methanol) with a Trans-Blot apparatus for 1h at 100 V (e.g., Bio-Rad). The proteins can be immunoblotted with specific antibodies. For example, membrane blotted with extracted proteins may be blocked with suitable buffers such as 3% solution of BSA or 3% solution of nonfat milk powder in TBST buffer (10 mM Tris-HCl, pH 8.6, 150 mM NaCl, 0.1% Tween 20) and incubated with monoclonal antibody specific to the polypeptide encoded by these gene variants. Unbound antibody is removed by washing with TBST for 5 X 1 minutes. Bound antibody may be detected using commercial ECL Western blotting detecting reagents.

The present invention also provides kits for diagnosing diseases associated with the presence of human KIP2V1 gene in a mammal, in particular, prostate cancer.

The kit of the present invention may be in any form suitable for providing (i) a pair of primers, (ii) a nucleic acid encoding KIP2V1, (iii) a nucleic acid fragment comprising nucleotides 242 to 247 of SEQ ID NO: 3, or (iv) the combination thereof for detecting the presence of human KIP2V1, together with written instructions for diagnosing the diseases associated with human KIP2V1 and/or prostate cancer. Examples include, but not limited to, various containers (e.g., bottles, cartons, blister packs, and ampoules) either accompanied by a package insert describing the detecting instructions, or wherein the instructions are printed on or affixed to the container.

The following examples are provided for illustration, but not for limiting the invention.

### EXAMPLES

### Analysis of Human EST Databases

Expressed sequence tags (ESTs) deposited at the National Center for Biotechnology Information (NCBI) was used as a source of EST sequence similarity search. Sequence comparisons against the nonredundant nucleotide and protein databases were performed using BLASTN and BLASTX programs (Altschul et al., (1997) Nucleic Acids Res. 25: 3389-3402; Gish and States, (1993) Nat Genet 3:266-272), at NCBI with a significance cutoff of p<10-10. No EST was found to represent KIP2V1 gene comprising the 32bp deletion from KIP2 nucleotides suggesting that KIP2V1 is a novel gene.

### Isolation of cDNA Clones

One cDNA clone showing sequence comprising 32bp deletion was isolated from a prostate cancer cDNA library and named KIP2V1. The inserts of this clone was subsequently excised in vivo from the λZAP Express vector using the ExAssist/XLOLR helper phage system (Stratagene). Phagemid particles were excised by coinfecting XL1-BLUE MRF' cells with ExAssist helper phage. The excised pBluescript phagemids were used to infect E. coli XLOLR cells, which lack the amber suppressor necessary for ExAssist phage replication. Infected XLOLR cells were selected using kanamycin resistance. Resultant colonies contained the double stranded phagemid vector with the cloned cDNA insert. A single colony was grown overnight in LB-kanamycin, and DNA was purified using a Qiagen plasmid purification kit.

### Full Length Nucleotide Sequencing and Database Comparisons

Phagemid DNA was sequenced using the Epicentre#SE9101LC SequiTherm EXCELTMII DNA Sequencing Kit for 4200S-2 Global NEW IR2 DNA sequencing system (LI-COR). Using the primer-walking approach, full-length sequence was determined. Nucleotide and protein searches were performed using BLAST against the non-redundant database of NCBI.

### In Silico Tissue Distribution (Northern) Analysis

The coding sequence for each cDNA clones was searched against the dbEST sequence database (Boguski et al., (1993) Nat Genet. 4: 332-3) using the BLAST algorithm at the NCBI website. ESTs derived from each tissue were used as a source of information for transcript tissue expression analysis. Tissue distribution for each isolated cDNA clone was determined by ESTs matching to that particular sequence variants (insertions or deletions) with a significance cutoff of p<10-10.

### RT-PCR expression Analysis

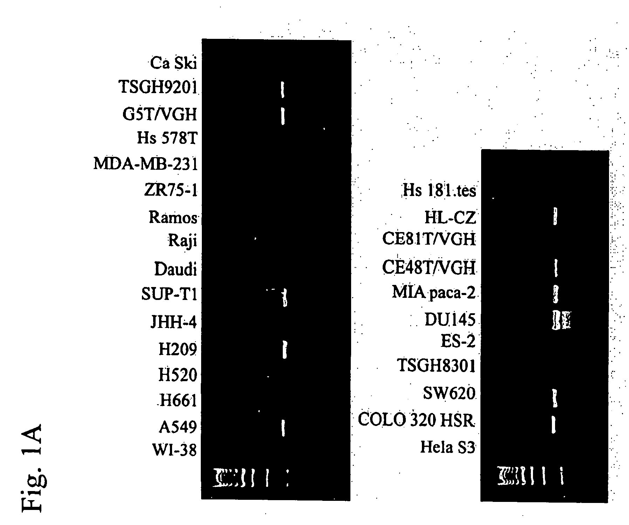

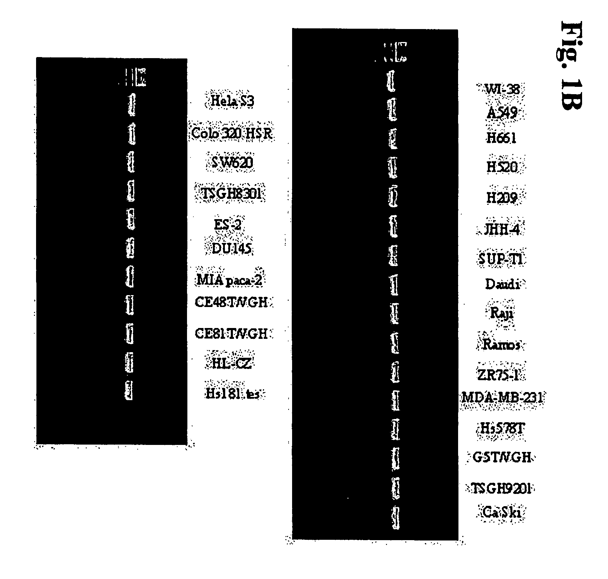

RT-PCR analysis of KIP2V1 was conducted in 27 human cell lines (WI38: lung, fetus; A549: lung cancer; H661: large cell carcinoma, lung; H520: squamous cell carcinoma, lung; H209: small cell carcinoma, lung; JHH-4: hepatoma; SUP-T1: T-cell lymophoblastic lymphoma; Daudi: Burkitt,s lymophoma; RAJI: Burkitt,s lymophoma; Ramo: Burkitt,s lymophoma; ZR75-1: breast carcinoma; MDA-MB-231: breast adenocarcinoma; Hs 578T: breast carcinoma; G5T/VGH: glioblastoma multiforme; TSGH9201: gastric carcinoma; Ca Ski: cervix epidermoid carcinoma; Hela S3: cervical epitheloid carcinoma; COLO 320 HSR: colon adenocarcinoma; SW620: adenocarcinoma; TSGH8301: urinary bladder carcinoma; ES-2: ovarian carcinoma; DU145: brain prostate carcinoma; MIA paca-2: pancreatic carcinoma; CE48T/VGH: esophagus epidermoid carcinoma; CE81T/VGH: esophagus carcinoma well differentiated aquamous; HL-CZ: promonocytic leukemia; and Hs 181.tes: normal testis). A pair of primers (forward: ACATCCTCAAGAGAATCCCTTC (SEQ ID NO: 7); reverse: GCTCACTAAGTCAGAGCTGGAT( SEQ ID NO: 8)) were designed for the RT-PCR analysis. The products of RT-PCR, electrophoresed in 1% agarose gel, show the sizes of approximately 235bp and 203bp (Fig. 1A). The alignment of the nucleotide sequences of LF (235bp) and SF (203bp) shown in Figs. 2A to 2D indicates that that LF matches to KIP2V and SF matches to KIP2V1. RT-PCR analysis of Glyceraldehyde phosphate dehydrogenase (GAPDH) was used as an internal control (Fig. 1B). Shown on the left side of Figs. 1A and 1B are 100 bp DNA ladder markers. It should be noted that KIP2V1 mRNA was only expressed in the cell line of prostate carcinoma suggesting that KIP2V1 may be used for diagnosing prostate cancer.

### REFERENCES

Adkins and Lumb, Intrinsic structural disorder and sequence features of the cell cycle inhibitor p57(Kip2). Proteins, 46:1-7 (2002).
Altschul et al., Gapped BLAST and PSI-BLAST: a new generation of protein database search programs, Nucleic Acids Res, 25: 3389-3402, (1997).
Ausubel et al., Current protocols in Molecular Biology, John Wiley & Sons, New York N.Y., ch. 9, 13, and 16, (1995).
Boguski et al., dbEST--database for "expressed sequence tags". Nat Genet. 4: 332-3, (1993).
Boyle et al., Incidence of prostate cancer will double by the year 2030: the argument for. Eur Urol. 29 Suppl 2:3-9, (1996).
Chung et al. Chromosome 11p15.5 regional imprinting: comparative analysis of KIP2 and H19 in human tissues and Wilms' tumors. Hum Mol Genet 5:1101-8 (1996).
Cote et al., Generation of human monoclonal antibodies reactive with cellular antigens, Proc Natl Acad Sci U S A 80: 2026-30 (1983).
Dauphinot et al. Analysis of the expression of cell cycle regulators in Ewing cell lines: EWS-FLI-1 modulates p57KIP2and c-Myc expression. Oncogene 20:3258-65, (2001)
de StGroth and Scheidegger, Production of monoclonal antibodies: strategy and tactics, J Immunol Methods 35:1-21, (1980).
Gimba and Barcinski, Molecular aspects of prostate cancer: implications for future directions. Int Braz J Urol, 29:401-410, (2003).
Gish and States, Identification of protein coding regions by database similarity search, Nat Genet, 3:266-272, (1993).
Harper and Elledge, Cdk inhibitors in development and cancer. Curr Opin Genet Dev 6:56-64, (1996).
Ito et al., Expression of p57/Kip2 protein in hepatocellular carcinoma. Oncology, 61:221-5, (2001)
Kozak, An analysis of 5'-noncoding sequences from 699 vertebrate messenger RNAs. Nucleic Acids Res, 15: 8125-48, (1987).
Kozak, An analysis of vertebrate mRNA sequences: intimations of translational control, J Cell Biol, 115: 887-903, (1991).
Kozbor et al., Specific immunoglobulin production and enhanced tumorigenicity following ascites growth of human hybridomas, J Immunol Methods, 81:31-42 (1985).
Lee and Yang, Negative regulators of cyclin-dependent kinases and their roles in cancers. Cell Mol Life Sci 58:1907-22, (2001).
Lee et al., Cloning of p57KIP2, a cyclin-dependent kinase inhibitor with unique domain structure and tissue distribution. Genes Dev 9:639-49, (1995).
Lindstedt et al., Determination of prostate-specific antigen in serum by immunoradiometric assay. Clin Chem. 36(1):53-58, (1990)
Liu et al., Silent mutation induces exon skipping of fibrillin-1 gene in Marfan syndrome. Nat Genet 16:328-9, (1997).
Lukas et al., Alternative and aberrant messenger RNA splicing of the mdm2 oncogene in invasive breast cancer. Cancer Res 61:3212-9, (2001).
Matsuoka et al. p57KIP2, a structurally distinct member of the p21CIP1 Cdk inhibitor family, is a candidate tumor suppressor gene. Genes Dev 9:650-62, (1995).
Oesterling et al., Prostate specific antigen in the preoperative and postoperative evaluation of localized prostatic cancer treated with radical prostatectomy. J Urol. 139(4):766-772, (1988).
Oya and Schulz, Decreased expression of p57(KIP2) mRNA in human bladder cancer. Br J Cancer 83:626-31, (2000).
Roberge et al., A strategy for a convergent synthesis of N-linked glycopeptides on a solid support. Science 269:202-4, (1995).
Sambrook, J. Cold Spring Harbor Press, Plainview N.Y., ch. 4, 8, and 16-17.
Sherr, Cancer cell cycles. Science 274:1672-7, (1996).
Siffert et al., Association of a human G-protein beta3 subunit variant with hypertension. Nat Genet, 18:45-8, (1998).
Stallings-Mann et al., Alternative splicing of exon 3 of the human growth hormone receptor is the result of an unusual genetic polymorphism. Proc Natl Acad Sci U S A 93:12394-9, (1996).
Stamey et al., Prostate-specific antigen as a serum marker for adenocarcinoma of the prostate. N Engl J Med. 317(15):909-16, (1987)
Watanabe et al. Suppression of cell transformation by the cyclin-dependent kinase inhibitor p57KIP2 requires binding to proliferating cell nuclear antigen. Proc Natl Acad Sci USA, 95:1392-1397, (1998).

## Claims

1. An isolated polypeptide comprising the amino acid sequence of SEQ ID NO: 4.

2. An isolated nucleic acid encoding the isolated polypeptide of Claim 1.

3. The isolated nucleic acid of Claim 2, which comprises the nucleotide sequence of SEQ ID NO: 3.

4. An expression vector comprising the nucleic acid of Claim 2.

5. A host cell transformed with the expression vector of Claim 4.

6. A method for producing the isolated polypeptide of Claim 1, which comprises the steps of:
(1) culturing the host cell of Claim 5 under a condition suitable for the expression of the polypeptide; and
(2) recovering the polypeptide from the host cell culture.

7. A method for diagnosing prostate cancer, which comprises detecting the presence of the isolated nucleic acid of Claim 2 or 3 from a sample obtained from a mammal.

8. The method of Claim 7, wherein the detection of the presence of the isolated nucleic acid of Claim 2 or 3 comprises the steps of:
(1) extracting total RNA from the sample obtained from the mammal;
(2) amplifying the RNA by reverse transcriptase-polymerase chain reaction (RT-PCR) with a pair of primers to obtain a cDNA sample comprising the nucleotides 242 to 247 of SEQ ID NO: 3; and
(3) detecting whether the cDNA sample is obtained.

9. The method of Claim 8, wherein one of the primers has a sequence comprising the nucleotides of SEQ ID NO: 3 containing nucleotides 242 to 247, and the other has a sequence complementary to the nucleotides of SEQ ID NO: 3 at any other locations downstream of nucleotide 247, or one of the primers has a sequence complementary to the nucleotides of SEQ ID NO: 3 containing nucleotides 242 to 247, and the other has a sequence comprising the nucleotides of SEQ ID NO: 3 at any other locations up stream of nucleotide 242.

10. The method of Claim 8, wherein one of the primers has a sequence comprising the nucleotides of SEQ ID NO: 3 upstream of nucleotide 242 and the other has a sequence complementary to the nucleotides of SEQ ID NO: 3 downstream of nucleotide 247.

11. The method of Claim 10, wherein the cDNA sample amplified from SEQ ID NO: 3 is 144 bp shorter than the cDNA sample amplified from human KIP2 gene or 32 bp shorter than the cDNA sample amplified from human KIP2V gene.

12. The method of any one of Claims 8 to 11 further comprising the step of detecting the amount of the amplified cDNA sample.

13. The method of Claim 7, wherein the detection of the presence of the isolated nucleic acid of Claim 2 or 3 comprises the steps of:
(1) extracting total RNA from the sample obtained from the mammal;
(2) amplifying the RNA by reverse transcriptase-polymerase chain reaction (RT-PCR) to obtain a cDNA sample;
(3) bringing the cDNA sample into contact with the isolated nucleic acid of Claim 2 or 3, or a nucleic acid fragment comprising nucleotides 242 to 247 of SEQ ID NO: 3; and
(4) detecting whether the cDNA sample hybridizes with the nucleic acid of Claim 2 or 3, or the nucleic acid fragment comprising nucleotides 242 to 247 of SEQ ID NO: 3.

14. The method of Claim 13 further comprising the step of detecting the amount of the hybridized sample.

15. A kit, which comprises:
(a) a pair of primers selected from the following pairs:
one of the primers has a sequence comprising the nucleotides of SEQ ID NO: 3 containing nucleotides 242 to 247, and the other has a sequence complementary to the nucleotides of SEQ ID NO: 3 at any other locations downstream of nucleotide 247,
one of the primers has a sequence complementary to the nucleotides of SEQ ID NO: 3 containing nucleotides 242 to 247, and the other has a sequence comprising the nucleotides of SEQ ID NO: 3 at any other locations up stream of nucleotide 242, and
one of the primers has a sequence comprising the nucleotides of SEQ ID NO: 3 upstream of nucleotide 242 and the other has a sequence complementary to the nucleotides of SEQ ID NO: 3 downstream of nucleotide 247;
an isolated nucleic acid of Claim 2 or 3;
a nucleic acid fragment comprising nucleotides 242 to 247 of SEQ ID NO: 3
or
a combination thereof; and
(b) printed instructions for detecting the presence of the polypeptide of Claim 1 and/or the nucleic acid of Claim 2 or 3.

## Patentansprüche

1. Isoliertes Polypeptid, welches die Aminosäuresequenz von SEQ ID Nr. 4 umfasst.

2. Isolierte Nukleinsäure, welche das isolierte Polypeptide nach Anspruch 1 kodiert.

3. Isolierte Nukleinsäure nach Anspruch 2, welche die Nukleotidsequenz von SEQ ID Nr. 3 umfasst.

4. Expressionsvektor, welcher die Nukleinsäure nach Anspruch 2 umfasst.

5. Wirtszelle, die mit dem Expressionsvektor nach Anspruch 4 transformiert ist.

6. Verfahren zum Herstellen des isolierten Polypeptids nach Anspruch 1, das die Schritte umfasst von:
(1) Kultivieren der Wirtszelle nach Anspruch 5 unter einer für die Expression des Polypeptids geeigneten Bedingung; und
(2) Gewinnen des Polypeptids aus der Wirtszellkultur.

7. Verfahren zum Diagnostizieren von Prostatakrebs, das ein Erfassen des Vorliegens der isolierten Nukleinsäure nach Anspruch 2 oder 3 aus einer von einem Säugetier erhaltenen Probe umfasst.

8. Verfahren nach Anspruch 7, wobei das Erfassen des Vorliegens der isolierten Nukleinsäure nach Anspruch 2 oder 3 die Schritte umfasst von:
(1) Extrahieren von Gesamt-RNA aus der von dem Säugetier erhaltenen Probe;
(2) Amplifizieren der RNA durch Reverse Transkriptase-Polymerasekettenreaktion (RT-PCR) mit einem Primerpaar, um eine cDNA-Probe zu erhalten, welche die Nukleotide 242 bis 247 von SEQ ID Nr. 3 umfasst; und
(3) Erfassen, ob die cDNA-Probe erhalten wurde.

9. Verfahren nach Anspruch 8, wobei einer der Primer eine Sequenz aufweist, welche die Nukleotide von SEQ ID Nr. 3, die Nukleotide 242 bis 247 enthalten, umfasst, und der andere eine Sequenz aufweist, die zu den Nukleotiden von SEQ ID Nr. 3 an beliebigen anderen Stellen stromabwärts von Nukleotid 247 komplementär ist, oder wobei einer der Primer eine Sequenz aufweist, die zu den Nukleotiden von SEQ ID Nr. 3, die Nukleotide 242 bis 247 enthalten, komplementär ist, und der andere eine Sequenz aufweist, welche die Nukleotide von SEQ ID Nr. 3 an beliebigen anderen Stellen stromaufwärts von Nukleotid 242 umfasst.

10. Verfahren nach Anspruch 8, wobei einer der Primer eine Sequenz aufweist, welche die Nukleotide von SEQ ID Nr. 3 stromaufwärts von Nukleotid 242 umfasst, und der andere eine Sequenz aufweist, welche zu den Nukleotiden von SEQ ID Nr. 3 stromabwärts von Nukleotid 247 komplementär ist.

11. Verfahren nach Anspruch 10, wobei die von SEQ ID Nr. 3 amplifizierte cDNA-Probe 144 bp kürzer ist als die vom KIP2-Gen des Menschen amplifizierte cDNA-Probe oder 32 bp kürzer ist als die vom KIP2V-Gen des Menschen amplifizierte cDNA-Probe.

12. Verfahren nach einem der Ansprüche 8 bis 11, das weiterhin den Schritt eines Erfassens der Menge der amplifizierten cDNA-Probe umfasst.

13. Verfahren nach Anspruch 7, wobei das Erfassen des Vorliegens der isolierten Nukleinsäure nach Anspruch 2 oder 3 die Schritte umfasst von:
(1) Extrahieren von Gesamt-RNA aus der von dem Säugetier erhaltenen Probe;
(2) Amplifizieren der RNA durch Reverse Transkriptase-Polymerasekettenreaktion (RT-PCR), um eine cDNA-Probe zu erhalten;
(3) In Kontakt bringen der cDNA-Probe mit der isolierten Nukleinsäure nach Anspruch 2 oder 3 oder einem Nukleinsäurefragment, welches Nukleotide 242 bis 247 von SEQ ID Nr. 3 umfasst; und
(4) Erfassen, ob die cDNA-Probe mit der Nukleinsäure nach Anspruch 2 oder 3 oder dem Nukleinsäurefragment, welches Nukleotide 242 bis 247 von SEQ ID Nr. 3 umfasst, hybridisiert.

14. Verfahren nach Anspruch 13, welches weiterhin den Schritt eines Erfassens der Menge der hybridisierten Probe umfasst.

15. Kit, welches umfasst:
(a) ein Primerpaar, welches aus den folgenden Paaren ausgewählt ist:
einer der Primer weist eine Sequenz auf, welche die Nukleotide von SEQ ID Nr. 3, die Nukleotide 242 bis 247 enthalten, umfasst, und der andere weist eine Sequenz auf, die zu den Nukleotiden von SEQ ID Nr. 3 an beliebigen anderen Stellen stromabwärts von Nukleotid 247 komplementär ist,
einer der Primer weist eine Sequenz auf, die zu den Nukleotiden von SEQ ID Nr. 3, die Nukleotide 242 bis 247 enthalten, komplementär ist, und der andere weist eine Sequenz auf, welche die Nukleotide von SEQ ID Nr. 3 an beliebigen anderen Stellen stromaufwärts von Nukleotid 242 umfasst, und
einer der Primer weist eine Sequenz auf, welche die Nukleotide von SEQ ID Nr. 3 stromaufwärts von Nukleotid 242 umfasst, und der andere weist eine Sequenz auf, die zu den Nukleotiden von SEQ ID Nr. 3 stromabwärts von Nukleotid 247 komplementär ist;
eine isolierte Nukleinsäure nach Anspruch 2 oder 3;
ein die Nukleotide 242 bis 247 von SEQ ID Nr. 3 umfassendes Nukleinsäurefragment
oder
eine Kombination davon; und
(b) gedruckte Anleitungen zum Erfassen des Vorliegens des Polypeptids nach Anspruch 1 und/oder der Nukleinsäure nach Anspruch 2 oder 3.

## Revendications

1. Un polypeptide isolé comprenant la séquence d'acides aminés SEQ ID NO : 4.

2. Un acide nucléique isolé codant le polypeptide isolé de la revendication 1.

3. L'acide nucléique isolé de la revendication 2 qui comprend la séquence de nucléotides SEQ ID NO : 3.

4. Un vecteur d'expression comprenant l'acide nucléique de la revendication 2.

5. Une cellule hôte transformée au moyen du vecteur d'expression de la revendication 4.

6. Une méthode de production du polypeptide isolé de la revendication 1 qui comprend les étapes de :
(1) cultiver la cellule hôte de la revendication 5 dans une condition appropriée à l'expression du polypeptide ; et
(2) récupérer le polypeptide à partir de la cellule hôte.

7. Une méthode pour diagnostiquer le cancer de la prostate qui comprend la détection de la présence de l'acide nucléique isolé de la revendication 2 ou 3 à partir d'un échantillon obtenu à partir d'un mammifère.

8. La méthode de la revendication 7 dans la quelle la détection de la présence de l'acide nucléique isolé de la revendication 2 ou 3 comprend les étapes de :
(1) extraire le RNA total à partir de l'échantillon obtenu à partir du mammifère ;
(2) amplifier le RNA par la réaction en chaîne de transcriptase-polymérase inverse (RT-PCR) au moyen d'une paire d'amorces pour obtenir un échantillon de cDNA comprenant les nucléotides 242 à 247 de SEQ ID NO : 3 ; et
(3) détecter si l'échantillon de cDNA est obtenu.

9. La méthode de la revendication 8 dans laquelle l'une des amorces comporte une séquence de nucléotides de SEQ ID NO : 3 contenant les nucléotides 242 à 247 et l'autre comporte une séquence complémentaire des nucléotides de SEQ ID NO : 3 à tout autre emplacement en aval du nucléotide 247, ou l'une des amorces comporte une séquence complémentaire des nucléotides de SEQ ID NO : 3 contenant les nucléotides 242 à 247 et l'autre comporte une séquence complémentaire des nucléotides de SEQ ID NO : 3 à tout autre emplacement en amont du nucléotide 242.

10. La méthode de la revendication 8 laquelle l'un des amorces comporte une séquence des nucléotides de SEQ ID NO : 3 en amont du nucléotide 242 et l'autre comporte une séquence complémentaire des nucléotides de SEQ ID NO : 3 en aval du nucléotide 247.

11. La méthode de la revendication 10 dans laquelle l'échantillon de cDNA amplifié à partir de SEQ ID NO : 3 est plus court de 144 bp que l'échantillon de cDNA amplifié à partir du gène humain KIP2 or plus court de 32 bp que l'échantillon de cDNA amplifié à partir du gène humain KIP2V.

12. La méthode de l'une quelconque des revendication 8 à 11 comprenant en outre l'étape de détection de la quantité de l'échantillon de cDNA amplifié.

13. La méthode de la revendication 7 dans laquelle la détection de la présence de l'acide nucléique isolé de la revendication 2 ou 3 comprend les étapes de :
(1) extraire le RNA total à partir de l'échantillon obtenu à partir du mammifère ;
(2) amplifier le RNA par la réaction en chaîne de transcriptase-polymérase inverse (RT-PCR) pour obtenir un échantillon de cDNA ;
(3) mettre l'échantillon de cDNA en contact avec l'acide nucléique isolé de la revendication 2 ou 3, ou un fragment d'acide nucléique comprenant les nucléotides 242 à 247 de SEQ ID NO : 3 ; et
(4) détecter si l'échantillon de cDNA s'hybride avec l'acide nucléique de la revendication 2 ou 3 ou le fragment d'acide nucléique comprenant les nucléotides 242 à 247 de SEQ ID NO :3.

14. La méthode de la revendication 13 comprenant en outre l'étape de détection de la quantité d'échantillon hybridé.

15. Un kit qui comprend :
(a) une paire d'amorces sélectionnées à partir des paires suivantes :
l'une des amorces comporte une séquence les nucléotides de SEQ ID NO : 3 contenant les nucléotides 242 à 247 et l'autre comporte une séquence complémentaire des nucléotides de SEQ ID NO : 3 à tout autre emplacement en aval du nucléotide 247,
l'une des amorces comporte une séquence complémentaire des nucléotides de SEQ ID NO: 3 contenant les nucléotides 242 à 247 et l'autre comporte une séquence complémentaire des nucléotides de SEQ ID NO : 3 à tout autre emplacement en amont du nucléotide 242, et
l'une des amorces comporte une séquence des nucléotides de SEQ ID NO : 3 en amont du nucléotide 242 et l'autre comporte une séquence complémentaire des nucléotides de SEQ ID NO : 3 en aval du nucléotide 247 ;
un acide nucléique isolé de la revendication 2 ou 3 ;
un fragment d'acide nucléique comprenant les nucléotides 242 à 247 de SEQ ID NO : 3 ou une combinaison de ceux-ci ; et
des instructions imprimées pour détecter la présence du polypeptide de la revendication 1 et / ou de l'acide nucléique de la revendication 2 ou 3.


### SEQUENCE LISTING

<110> Dai, Ken-Shwo
<120> HUMAN KINASE INTERACTING PROTEIN 2 (KIP2)-RELATED GENE VARIANT (KIP2V1) ASSOCIATED WITH PROSTATE CANCER
<130> U 015772-6
<140>
   <141>
<160> 8
<170> PatentIn version 3.1
<210> 1
   <211> 1123
   <212> DNA
   <213> Homo sapiens
<400> 1
   ggcggccacc atggggaaca agcagaccat cttcaccgaa gagcagctag acaactacca      60
   ggactgcacc ttcttcaata agaaggacat cctcaagaga atcccttcaa agaaaggatc     120
   gtggcggcgt tttccgagga tggtgagggg aacctcactt tcaacgactt tgtggacatg     180
   ttttccgtgc tctgcgagtc ggctccccga gagctcaagg caaactatgc cttcaagatc     240
   tatgacttca acactgacaa cttcatctgc aaggaggacc tggagctgac gctggcccgg     300
   ctcactaagt cagagctgga tgaggaggag gtggtgcttg tgtgcgacaa ggtcattgag     360
   gaggctgact tggatggtga cggcaagctg ggctttgctg acttcgagga catgattgcc     420
   aaggcccctg acttcctcag cactttccac atccggatct gaggacactg ccgaggctgt     480
   aggggcctag aagtccacca tcctgccctg cagtcacatg ggtgtggcct ccaagctccc     540
   caggaaagca gtggcagcct ctggggttta caccacaaat atatcctgtg gccctttcag     600
   caaaaaaaaa accttaacca ggaagggggc ctgtgaaggt taggaccctt cccacagccc     660
   cgctgtggtc cagccccggg acccatggcc tctctccagg ccctccgcac cccgccccat     720
   gccccagtcc tttcctactc cagaaatgct ccccaccccc ccaagaggga aaagcagata     780
   acccaacagg aggtggtggg gtctgacgtg tccaagtgct ggcagacacc ctggtcaccc     840
   aggacagagc ggaaaaaaaa aaaagaggtc agggttttaa cgagctatgc aatctttttc     900
   caaaacccaa ggttgggctg cttcccaccc ctgcctgttc cccttctccc ggcctccttc     960
   acaatgtgaa gctgtgggtg cagtggcgcc aagggctctt gctcctgtct ctgcctctgg    1020
   gtcatgagta ccaccctgcc tgcctcccca acaccgtgga atcctcactg gtgtgctgtc    1080
   cacagatttg tgaactcctg gtagtaaaac acttttgcat cca                      1123
<210> 2
   <211> 92
   <212> PRT
   <213> Homo sapiens
<400> 2
   Met Gly Asn Lys Gln Thr Ile Phe Thr Glu Glu Gln Leu Asp Asn Tyr
   1               5                   10                  15
   Gln Asp Cys Thr Phe Phe Asn Lys Lys Asp Ile Leu Lys Arg Ile Pro
               20                  25                  30
   Ser Lys Lys Gly Ser Trp Arg Arg Phe Pro Arg Met Val Arg Gly Thr
           35                  40                  45
   Ser Leu Ser Thr Thr Leu Trp Thr Cys Phe Pro Cys Ser Ala Ser Arg
       50                  55                  60
   Leu Pro Glu Ser Ser Arg Gln Thr Met Pro Ser Arg Ser Met Thr Ser
   65                  70                  75                  80
   Thr Leu Thr Thr Ser Ser Ala Arg Arg Thr Trp Ser
                   85                  90
<210> 3
   <211> 1091
   <212> DNA
   <213> Homo sapiens
<400> 3
<210> 4
   <211> 139
   <212> PRT
   <213> Homo sapiens
<400> 4
   Met Gly Asn Lys Gln Thr Ile Phe Thr Glu Glu Gln Leu Asp Asn Tyr
   1               5                   10                  15
   Gln Asp Cys Thr Phe Phe Asn Lys Lys Asp Ile Leu Lys Arg Ile Pro
               20                  25                  30
   Ser Lys Lys Gly Ser Trp Arg Arg Phe Pro Arg Met Val Arg Gly Thr
           35                  40                  45
   Ser Leu Ser Thr Thr Leu Trp Thr Cys Phe Pro Cys Ser Ala Ser Arg
       50                  55                  60
   Leu Pro Glu Ser Ser Arg Gln Thr Met Pro Ser Arg Ser Met Asp Leu
   65                  70                  75                  80
   Glu Leu Thr Leu Ala Arg Leu Thr Lys Ser Glu Leu Asp Glu Glu Glu
                   85                  90                  95
   Val Val Leu Val Cys Asp Lys Val Ile Glu Glu Ala Asp Leu Asp Gly
               100                 105                 110
   Asp Gly Lys Leu Gly Phe Ala Asp Phe Glu Asp Met Ile Ala Lys Ala
           115                 120                 125
   Pro Asp Phe Leu Ser Thr Phe His Ile Arg Ile
       130                 135
<210> 5
   <211> 11
   <212> DNA
   <213> Artificial sequence
<220>
   <221>
   <222>
   <223> A polynucleotide sequence provided to illustrate what is meant by the term "complement"
<400> 5
   atggacttac t 11
<210> 6
   <211> 11
   <212> DNA
   <213> Artificial sequence
<220>
   <221>
   <222>
   <223> A polynucleotide sequence provided to illustrate what is meant by the term "complement"
<400> 6
   agtaagtcca t 11
<210> 7
   <211> 22
   <212> DNA
   <213> Homo sapiens
<220>
   <221>
   <222>
   <223> Primer
<400> 7
   acatcctcaa gagaatccct tc 22
<210> 8
   <211> 22
   <212> DNA
   <213> Homo sapiens
<220>
   <221>
   <222>
   <223> Primer
<400> 8
   gctcactaag tcagagctgg at 22